# EUROPEAN PATENT APPLICATION

(11) **EP 1 912 069 A2**
(43) Date of publication of application: **16.04.2008**
(21) Application number: 08000542.4
(22) Date of filing: 05.06.2003
(51) Int. Cl.: G01N 33/566, G01N 33/50, G01N 33/74, C07K 2/00, C07K 14/475, C07K 14/705, C07K 14/715, C12N 5/10, C12N 15/11, C12N 15/12, C12N 15/62, C12N 15/63

(54) **Method to screen ligands using eukaryotic cell display**

(30) Priority: 05.06.2002 US 386584 P
(62) Divisional of application: 03736907.1
(71) Applicant: Sopherion Therapeutics, Inc., New Haven, CT 06511 (US)
(72) Inventor: Ashkar, Samy, Boston, MA 02115 (US)
(74) Representative: Cripps, Joanna Elizabeth

(57) **Abstract**

Methods and compositions for the screening and identification of ligands that are expressed and displayed on the outer surface of eukaryotic cells and screening for binding of the ligand to a receptor domain of a chimeric fusion receptor protein which is also expressed and displayed on the surface of the cells have been developed. Binding to the receptor domain activates, inhibits, or modulates the enzymatic function of the receptor intracellular domain of the fusion protein.

## Description

### Cross Reference to Related Application

Priority is claimed to U.S. Provisional Application Serial No. 60/386,584 filed on June 5, 2002.

### BACKGROUND OF THE INVENTION

The present invention is generally in the field of high throughput peptide screening, and in particular relates to eukaryotic cell display technology for the generation and screening of random peptides.

The interaction between cognate proteins in receptor-ligand complexes, enzyme substrate reactions and antibody-antigen binding reactions has furthered the understanding of the molecular interactions required to effect a response in a wide range of processes. The search for new peptide molecules which can bind to selected targets and effectively modulate a particular biological process is at the forefront of agricultural, biological, and medicinal research.

There are several reported methods that use peptides or nucleotides to develop libraries of potential receptor, enzyme, or antibody interacting peptides. Over the course of the last two decades these libraries have been incorporated into systems that allow the expression of random peptides on the surface of different phage or bacteria. For example, many publications have reported the use of phage display technology to produce and screen libraries of polypeptides for binding to a selected target. See, e.g, Cwirla et al., Proc. Natl. Acad. Sci. USA 87, 6378-6382 (1990); Devlin et al., Science 249, 404-406 (1990), Scott & Smith, Science 249, 386-388 (1990); U.S. Patent No. 5,571,698 to Ladner *et al.* A basic concept of phage display methods is the establishment of a physical association between DNA encoding a polypeptide to be screened and the target polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target. These phage are enriched by affinity screening to the target. The identity of polypeptides displayed from these phage can be determined from their respective genomes. Using these methods a polypeptide identified as having a binding affinity for a desired target can then be synthesized in bulk by conventional means.

In addition to providing a method for selecting peptides that interact with target molecules, phage display has been used to direct filamentous phage to target cells using peptides, genetically fused to phage coat proteins, that bind integrin proteins on the surface of mammalian cells. This method of phage display has had a profound influence on gene therapy applications and their attempts to target cells in a specific manner.

Another approach to obtaining surface expressed foreign proteins has been the use of bacterial native membrane proteins as carriers for foreign protein. In general, many attempts to develop methods of anchoring proteins on a bacterial surface have focused on fusion of the desired recombinant polypeptide to a native protein that is normally exposed on the cell's exterior with the hope that the resulting hybrid will also be localized on the surface. However, in most cases, the foreign protein interferes with localization, and thus, the fusion protein is unable to reach the cell surface. These fusions either end up at incorrect cellular locations or become anchored in the membrane with a secreted protein domain facing the periplasm. See Murphy, et al., J. Bacteriol., 172:2736 (1990).

Recent advances in bacterial display methods have circumvented this problem by using fusion proteins comprising pilin protein (TraA) or a portion thereof and a heterologous polypeptide displaying the library peptide on the outer surface of the bacterial host cell capable of forming pilus. See U.S. Patent. No. 5,516,637 to Huang et al. The pilus is anchored to the cell surface of the bacteria and is naturally solvent exposed.

Alternatively, the FLITRX^{™} (Invitrogen Corp.) random peptide library uses the bacterial flagellar protein, FliC, and thioredoxin, TrxA, to display a random peptide library of dodecamers on the surface of *E.coli* in a conformationally constrained manner. See Lu et al., BioTechnology, 13:366 (1995). These systems have been applied to antibody epitope mapping, the development and construction of live bacterial vaccine delivery systems, and the generation of whole-cell bio-adsorbants for environmental clean-up purposes and diagnostics. Peptide sequences that bind to tumor specific targets on tumor derived epithelial cells have also been identified using the FLITRX^{™} system. See Brown et al., Annals of Surgical Oncology, 7(10):743 (2000).

Although the phage and bacterial display systems have provided unique routes to elucidating new peptides which can bind target molecules with new or enhanced binding properties, there are several important limitations that need to be considered. Bacterial and phage display systems rely upon cumbersome and time consuming techniques in order to keep conditions optimal for cell growth and cell viability. Bacterial cells are relatively large and care must be taken while screening for target interacting peptides. Affinity chromatography is a common method used to separate non-binding peptides from binding peptides and care must be taken to prevent plugging and the non-specific retention of bacteria in the column. Candidate peptide displaying phage are generally amplified or propagated and therefore require the use of the cellular transcriptional, translational, and replication machinery of bacteria to synthesize the packaging proteins of the phage as well as the peptide of interest. Infecting bacterial cells, harvesting the phage, and re-infecting several rounds is very time consuming. The bacterial cell display system also requires optimal growth conditions to ensure safe passage of the plasmid encoded peptide from generation to generation and for subsequent re-screening.

A display system that provides for efficient use of endogenous cellular machinery for the *in vivo* manipulation and humanization of proteins is desired. Such use will alleviate any further genetic manipulation of the host strain for post-translational modifications that may be required to generate sufficient membrane targeting signals and/or generate desired glycosylation or lipid modification of the expressed protein or peptide.

A display system that allows one to easily assess the interaction between peptide and target molecule without cumbersome and time consuming techniques such as affinity chromatography is desired.

It is therefore an object of this invention to provide an effective and rapid method of the systematic preparation of novel peptide substrates and to address the shortcomings inherent in the phage and bacterial display methods currently practiced in the art.

### BRIEF SUMMARY OF THE INVENTION

Methods and compositions for the identification of ligands that target specific receptors are disclosed. The ligands are expressed and displayed on the outer surface of cells and screened for *via* binding of the ligand to a receptor domain of a chimeric fusion receptor protein on the surface of the same cells. Binding to the receptor domain activates the cell receptor intracellular domain of the fusion protein, thereby activating, inhibiting, or modulating an intracellular signaling pathway.

Any potential binding domain (receptor domain) may be fused to the intracellular domain of the receptor (directly or indirectly *via* a genetic linker). All that is required is that the binding domain "transmit" the appropriate signal to the intracellular domain, resulting in autophosphorylation or proper intracellular enzymatic activity. Non-binding ligands may result in non-signaling *via* the chimeric receptor, or default signaling; depending upon the receptor and the pathway it activates, inhibits, or modulates.

Optionally, the interaction between ligand and receptor may take place in the cytoplasm, wherein the deletion of transmembrane domain of a receptor, that is normally present on a cell surface, will result in a soluble or cytoplasmic receptor. The ligand may be expressed as a soluble protein (remains in the cytoplasm), wherein it binds to the receptor.

An example of the foregoing description is the identification of ligands that are expressed and displayed on the outer surface of endothelial cells. For example, binding of a ligand to the receptor domain of a chimeric receptor that contains a vascular endothelial cell receptor intracellular domain, would result in the inhibition of apoptosis, or cell death, and allow the cell to grow. Cell growth is easily assayed *via* any number of well-known techniques. Non-binding ligands, and therefore non-signaling of VEGF chimera receptors, result in cell death, or apoptosis.

### DETAILED DESCRIPTION OF THE INVENTION

### I. System for Screening

Methods for selecting oligonucleotides and peptide ligands that bind to molecules of interest, and generating and screening large cell display libraries for peptides with desired functional binding characteristics have been developed in a eukaryotic cell system. These methods allow for one to take advantage of natural intracellular processes that occur in eukaryotic cells but do not naturally occur in bacterial cells or phage systems. For example, *de novo* synthesized proteins may undergo further processing in mammalian cells, known as post-translational modification. In particular, sugar residues may be added enzymatically, a process known as glycosylation. The resulting proteins bearing covalently linked oligosaccharide side chains are known as glycosylated proteins or glycoproteins. Bacteria typically do not glycosylate proteins; in cases where glycosylation does occur it usually occurs at nonspecific sites in the protein (Moens and Vanderleyden, Arch. Microbiol. 1997 168(3):169-175). The production of glycoproteins more closely resembling human forms of proteins is a distinct advantage of using the endothelial cell display system described herein. Furthermore, modification required for the attachment of each displayed peptide and receptor to the membrane of the same cell may be accomplished by in vivo modification systems that are inherently a part of the secretory pathways utilized by eukaryotic cells (for example, lipid and phospholipid modifying enzymes).

Once expressed, and preferably displayed on the surface of the eukaryotic cell, the ligands are screened for binding to a receptor domain of a chimeric fusion receptor protein that is expressed and displayed on the surface of the same cell. Binding to the receptor domain activates the intracellular domain of the fusion protein, thereby activating, inhibiting, or modulating an intracellular signaling pathway. The enzymatic activity of any component in the signaling pathway may be assayed by methods known in the molecular biological field. Alternatively, the cellular phenotype, or physiological state, may be readily observed upon receptor binding. While it is preferable that the cell is mammalian, any eukaryotic cell may be used in the methods described herein. For example, plant cells, animal cells and yeast cells may all be used.

### A. Chimeric Receptors to be Expressed and Displayed

The term "chimeric receptor" refers to a receptor having amino acid sequences derived from at least two different proteins. For example, a chimeric receptor comprising the extracellular ligand binding domain of one receptor, such as the erythropoetin binding domain of the erythropoetin receptor, and having the intracellular domain of a VEGF receptor. Binding of ligand to the extracellular domain stimulates the VEGF receptor's intracellular domain, resulting in cell dedifferentiation and the formation of a new vasculature by a process known as pathological angiogenesis, or pathoangiogenesis. The VEGF intracellular autophosphorylation domain is genetically fused to the extracellular ligand binding domain. It is to be understood that any intracellular domain may be used in conjunction with the herein described method. For example, the intracellular domain may have enzymatic activity and/or may serve as a binding site for additional proteins. Frequently, the intracellular domain of transmembrane proteins serves both roles. For example certain receptor tyrosine kinases have both protein kinase activity and SH2 domains. In addition, autophosphorylation of tyrosines on the receptor molecule itself, creates binding sites for additional SH2 domain containing proteins. It is preferred that the intracellular domain govern the development of an assayable phenotype, for example, cell growth or cell apoptosis. Alternatively, the intracellular domain's enzymatic function, itself, may be assayed *via* well known molecular biological techniques.

DNA encoding the various domains of the receptor to be used as a template, or starting composition for construction of the chimera, such as the intracellular, transmembrane, and/or extracellular domains can be deleted and or replaced by DNA encoding corresponding domains from other receptors. For example, the deletion of transmembrane domain will result in a soluble, or cytoplasmic receptor. DNA encoding unrelated amino acid sequences may also be fused to the DNA encoding some or all of the receptor, thereby producing a chimeric receptor molecule. Any potential binding molecule domain may be fused to the intracellular domain of the receptor (directly or indirectly). All that is required is that the binding domain "transmit" the appropriate signal to the intracellular domain, resulting in autophosphorylation or proper intracellular enzymatic activity. The resulting chimera receptor may be membrane bound, may be soluble (i.e. cytoplasmic), or may be secreted. For example, chimeras constructed from a protein receptor sequence linked to an appropriate immunoglobulin constant domain sequence (immunoadhesions) are known in the art. Immunoadhesions reported in the literature include fusions of the T-cell receptor (Gascoigne et al., Proc. Natl. Acad. Sci. USA 84:2936-2940; 1987), CD4 (Capon et al., Nature 337:525-531; 1989), L-selectin (homing receptor) (Watson et al., J. Cell. Biol. 110:2221-2229; 1990), CD44 (Aruffo et al., Cell 61:1303-1313; 1990), CD28 and B7 ( Linsley et al., J. Exp. Med. 173:721-730; 1991), CTLA-4 (Linsley et al., J. Exp. Med. 174:561-569; 1991), CD22 (Stamenkovic et al., Cell 66:1133-1144; 1991), TNF receptor (Ashkenazi et al., Proc. Natl. Acad. Sci. USA 88:10535-10539; 1991) and IgE receptor alpha (Ridgway et al., J. Cell. Biol. 115:abstr. 1448; 1991).

Receptor protein fragments for the construction of chimeras are prepared by well known genetic and recombinant methods. For example, DNA encoding any of the natural VEGF receptors described above may be obtained from vascular endothelial cells by:
(a) preparing a cDNA library from these cells,
(b) conducting hybridization analysis with labeled DNA encoding the VEGF receptor or fragments thereof to detect clones in the library containing homologous sequences, and
(c) analyzing the clones by restriction enzyme analysis and nucleic acid sequencing to identify full-length clones. If full length clones are not present in a cDNA library, then appropriate fragments may be recovered from the various clones using the nucleic acid and amino acid sequence information known for the VEGF receptors and ligated at restriction sites common to clones to assemble a full length clone encoding the receptor. Alternatively, genomic libraries may provide the desired DNA. Given the above detailed description of the known VEGF receptors, one may utilize primers to known sequences in combination with polymerase chain reaction to isolate the desired VEGF receptor.

The term "fusion protein" as used herein refers to a chimeric protein construct that is the result of combining two or more domains and/or linker regions from different proteins for the purpose of combining in one single polypeptide chain functions and recognition properties normally associated with two or more polypeptides. This is most often accomplished by the adjacent molecular cloning of the nucleotide sequences encoding for the desired protein domains to result in the creation of a new polynucleotide sequence that codes for the desired protein. Alternatively, creation of a fusion protein may be accomplished by chemically joining two proteins together. For example, DNA encoding the various domains of the VEGF receptor, such as the intracellular, transmembrane, and/or various Ig-like domains can be deleted and or replaced by DNA encoding corresponding domains from other receptors. DNA encoding unrelated amino acid sequences may also be fused to the DNA encoding some or all of the VEGF receptor, thereby producing a chimeric VEGF receptor molecule.

The term "protein domain" as used herein refers to a region of a protein that can fold into a stable three-dimensional structure, often independently of the rest of the protein, and which is endowed with a particular function. This structure may maintain a specific function associated with the domain's function within the original protein including enzymatic activity, creation of a recognition motif for another molecule, or provide necessary structural components for a protein to exist in a particular environment of proteins, both within a protein family and within related protein superfamilies, protein domains can be evolutionarily conserved regions.

The term "linker region" or "linker domain" or similar such descriptive terms as used herein refers to stretches of polynucleotide or polypeptide sequence that are used in the construction of a cloning vector of fusion protein. Functions of a linker region can include introduction of cloning sites into the nucleotide sequence, introduction of a flexible component or space-creating region between two protein domains, or creation of an affinity tag for specific molecule interaction. A linker region may be introduced into a fusion protein without a specific purpose, but as a compromise that results from choices made during cloning.

The term "cloning site" or "polycloning site" as used herein refers to a region of the nucleotide sequence contained with a cloning vector or engineered with a fusion protein that has one or more available restriction endonuclease recognition sequences. Adequate manipulation of restriction endonuclease sites allows one to clone in tandem two or more nucleotide sequences so that the respective encoded protein domains are translated in frame relative to a particular start codon, thus yielding a desired protein product after transcription and translation. These nucleotide sequences can then be introduced into other cloning vectors, used to create novel fusion proteins, or used to introduce specific site-directed mutations. It is well known in the art that cloning sites can be engineered at a desired location by silent mutations, conserved mutation, or introduction of a linker region that contains desired restriction enzyme consensus sequences. It is also well known by those in the art that the precise location of a cloning site can be flexible so long as the desired function of the protein or fragment thereof being cloned is maintained.

### AI. "Templates" for Use in Generating Domains of the Chimeric Receptor (Input Domain, Output Domain and Transmembrane Domain)

### Erythropoietin (EPO) Receptors (having both input ligand binding domain and output signaling domain)

The EPO receptor is a single transmembrane (Mr 70,000) protein that is activated *via* EPO-induced dimerization, with a single EPO molecule bridging EPO receptor pairs. EPO-induced receptor dimerization leads to the dimerization and cross-phosphorylation of one or more members of the JAK family of cytoplasmic tyrosine kinases. Ligand induced receptor dimerization leads to the activation of JAKs, rapid tyrosine-phosphorylation of the cytoplasmic domains, and subsequent recruitment of various signaling proteins. Among these signaling proteins are a complex set of SH2 domain-encoding signal transduction factors. These include latent transcription factor (STAT5), phospholipid modifying enzymes (P13K, PLC-gamma 1, SHIP), regulators of Ras and MAP kinase signaling, tyrosine phosphatases (SHP1 and SHP2), suppressors of cytokine signaling (Cis, SOCS3), and Src-family kinases. EPO receptors complexes also modulate p38 and JNK kinases, as well as a variety of immediate response genes *via* Jak2-dependent routes. SHP-1 proteins function by down regulating the EPO induced signal and thus hematopoeisis. SHP-1 inhibition results in EPO responsive cells to become hypersensitive to EPO. This inhibition can be accomplished by targeting several receptors on the4 BFU-E or CFU-E cells. These include IL-3 receptor, angiotensin receptor and the alpha4beta1 integrin. Molecules that can target and activate these receptors are isolated using the presently disclosed methods.

### VEGF and Natural VEGF Binding Receptors (having both input ligand binding domain and output signaling domain)

The biological function of VEGF is mediated through binding to two high affinity receptors which are selectively expressed on endothelial cells during embryogenesis (Millauer et al., (1993) Cell 72:835-838) and VEGF related pathologies (tumor formation). VEGF receptors include the human kinase domain receptor (KDR), described in U.S. Patent No. 5,713,380; its murine analog flk-1, sequenced by Mallhews (1991) Proc. Natl. Acad. Sci. USA, 88:9026-9030; U.S. Patent No. 5,270,458 and the Fsm-like tyrosine kinase (Flt-1) (Shibuya et al., (1990) Oncogene 5:519-524). All of them are class III tyrosine kinases (Vaisman et al., 1990: J. Biol. Chem. 265, 19461-19466; Kaipainen et al., (1993) J. Exp. Med. 178:2077-2088). Studies in mice have shown that the expression of KDR reaches the highest levels during embryonic vasculogenesis and angiogenesis (Millauer et al., 1993 Cell 72:835-838). In contrast, only low levels of mRNA for Flt-1 were found during fetal growth and moderate levels during organogenesis, but high levels in newborn mice (Peters et al., 1993 Proc. Natl. Aca. Sci. U.S.A. 90(16):7533-7). Experiments with knockout mice deficient in either receptor revealed that KDR is essential for the development of endothelial cells, whereas Flt-1 is necessary for the organization of embryonic vasculature (Fong et al., 1995 Dev. Dyn. 203(1):80-92; Shalaby et al., 1995 Nature 376 (6535:62-6). KDR and Flt-1, each about 1300 amino acid residues long, are composed of 7 extracellular Ig-like domains containing the ligand-binding region, a single short membrane-spanning sequence, and an intracellular region containing tyrosine kinase domains. The amino acid sequences of KDR and Flt-1 are roughly 45% identical to each other. Flt-1 has the higher affinity for VEGF (K_{D}=10-20 pM) compared to 75-125 pM for the KDR receptor. VEGF binding to KDR but not Flt-1 elicits an efficient (ED50.about.0.1-1 ng/ml) DNA synthetic and chemotactic endothelial cell response. Activation of Flt-1 receptor by VEGF might modulate the interaction of endothelial cells with each other or the basement membrane on which they reside.

The Flt-1 receptor mRNA can be spliced to generate forms encoding either the full-length membrane-spanning receptor or a soluble form, denoted sFlt-1. Pure sFlt-1 retains its specific high affinity binding for VEGF and fully inhibits VEGF-stimulated endothelial cell mitogenesis by dominant negative mechanism.

Domain deletion studies on Flt-1 receptor have shown that the ligand binding function resides within the first three domains (Barleon et al., (1997) J. Biol. Chem. 272:10382-1038; Cunningham et al., (1997) Biochim. Biophys. Res. Commun. 231 (3): 596-599), and domain 4 is required to efficiently couple ligand binding to signal transduction by means of direct receptor-receptor contacts (Barleon et al., (1997) J. Biol. Chem. 272:10382-10388). The crystal structure of the complex between VEGF and the second domain of Flt-1 showed domain 2 in a predominantly hydrophobic interaction with the "poles" of VEGF dimer (Wiesmann et al., (1997) Cell 91:695-704). Deletion experiments on KDR demonstrated that only domain 2 and 3 are critical for ligand binding (Fuh et al., (1998) J. Biol Chem. 1998; 273 (18):11197-204).

Endothelial cells also contain another type of VEGF receptor, Neuropilins (NP), possessing a lower mass than either VEGFR2 or VEGFR1 (Gluzman-Poltorak Z., et al., (2000) J. Biol. Chem., 275(24):18040-5; WO Patent 0002/3565A2). It was subsequently found that these smaller VEGF receptors of endothelial cells are isoform specific receptors that bind VEGF 165 but not VEGF121 (Gluzman-Poltorak Z, et al., (2000) J. Biol. Chem., 275(38):29922). Unusually large amounts of these isoform-specific receptors were found in several types of prostate and breast cancer cell lines (Miao, H. Q., et al., (2000) FASEB J., 14(15):2532-9). Neuropilin-1 is likely to play an important role in the development of the cardiovascular system. Gene disruption studies have indicated that np-1 participates in embryonic vasculogenesis and angiogenesis and is involved in the maturation of blood vessels, since mouse embryos lacking a functional np-I gene die because their cardiovascular system fails to develop properly (Kawasaki, T., et al., (1999) Neurobiol. 39(4):579-89). Subsequent experiments have shown that NP-1 also serves as a receptor for the heparin-binding form of placenta growth factor (PlGF), PlGF-2, and for VEGF-B (VEGF-B is described in International Patent Application PCT/US96/02957 (WO 96/26736) and in U.S. Pat. Nos. 5,840,693 and 5,607,918 by Ludwig Institute for Cancer Research and The University of Helsinki).

Another endothelial cell-specific receptor tyrosine kinase, Flt4 (or VEGFR-3) is expressed in venous and lymphatic endothelia in the fetus, and predominantly in lymphatic endothelia in the adult (Kaipainen et al., Cancer Res., 1994, 54:6571-6577; Proc. Natl. Acad. Sci. USA, 1995, 92:3566-3570). The ligand VEGF-C (or VEGF-2) (described in Joukov et al., EMBO J., 15: 290-298, 1996, Lee et al., Proc. Natl. Acad. Sci. USA, 93: 1988-1992, 1996, and U.S. Pat. Nos. 5,932,540 and 5,935,540 by Human Genome Sciences, Inc.) has been shown to be the ligand for Flt4, and also activates KDR (Joukov et al., EMBO J., 15: 290-298, 1996).

### Transmembrane Domains

Transmembrane regions of proteins are highly hydrophobic or lipophilic domains that are the proper size to span the lipid bilayer of the cellular membrane, thereby anchoring proteins, peptides, or receptors in the cell membrane. Other cell surface molecules are anchored by lipid modification (often a covalently attached lipid; either a fatty acid chain or prenyl group), or by phospholipid anchors (for example, phosphatidylinositol) in the non-cytoplasmic monolayer of the lipid bilayer. Many proteins are attached *via* noncovalent interactions with other membrane proteins. The methods and compositions disclosed herein utilize the well characterized endogenous protein modification systems of mammalian cells to generate membrane anchored molecules.

Transmembrane proteins may contain from one to many transmembrane domains. For example, receptor tyrosine kinases, certain cytokine receptors, receptor guanylyl cyclases and receptor serine/threonine protein kinases contain a single transmembrane domain. However, various other proteins including channels and adenylyl cyclases contain numerous transmembrane domains. Many important cell surface receptors are classified as "seven transmembrane domain" proteins, as they contain 7 membrane spanning regions. Important transmembrane protein receptors include, but are not limited to insulin receptor, insulin-like growth factor receptor, human growth hormone receptor, glucose transporters, transferrin receptor, epidermal growth factor receptor, low density lipoprotein receptor, epidermal growth factor receptor, leptin receptor, interleukin receptors, e.g. IL-1 receptor, IL-2 receptor, etc. Characteristics of transmembrane domains include approximately 20 consecutive hydrophobic amino acids that may be followed by charged amino acids. Therefore, upon analysis of the amino acid sequence of a particular protein, the localization and number of transmembrane domains within the protein may be predicted.

### B. Ligand/Ligand Library

Expression of a ligand in the host endothelial cell and proper localization to the outer surface of the plasma membrane is central to the disclosed method. The method consists of first constructing a ligand/substrate peptide library wherein the library may consist of a retroviral expression vector which includes a transcriptional regulatory element operably linked to a gene fusion, where the gene fusion includes (there are many examples of retroviral expression vectors, for example see U.S. Patent No. 6,255,071):
(i) a first gene encoding at least a portion of a endothelial cell plasma membrane protein, or at least sequences required for proper modification of the gene product for plasma membrane attachment or insertion; and
(ii) a second gene or oligonucleotide encoding a potential "substrate" peptide for a receptor, wherein the receptor is defined, in part, by a ligand binding domain fused to an intracellular signaling domain (either directly, or indirectly *via* a linker as described above), for example, a VEGF signaling domain. The 3' end of the first gene is linked to the 5' end of the second gene or oligonucleotide, thereby forming a chimeric gene. The chimeric gene encodes a chimeric protein. The linkage between the first and second gene may be direct, or indirect *via* a linker molecule or oligonucleotide. Such a linker molecule may provide for flexibility and "reach" so that the expressed peptide may contact the receptor of interest. The second gene or oligonucleotide is obtained from a library of random oligonucleotides constructed by degenerate polymerase chain reaction (PCR), a method well known within the art, or other amplification method. In certain embodiments, it is desirable that the first gene encodes an outer membrane protein, or portion thereof, amenable to fusing large oligonucleotides.

It is optional that the peptides or ligands be soluble, or cytoplasmic. When cytoplasmic, the ligand bound chimera receptor is still able to signal the inhibition of apoptosis *via* the VEGF domain.

Once constructed, the oligonucleotide library harboring retrovirus is used to infect the endothelial strain of choice and the oligonucleotide expressed. Alternatively, any other commonly used method to stably introduce nucleic acid into cells may be used, including electroporation.

Expression vectors (for receptor and ligand expression in the same cell) to be used in eukaryotic cells, and most preferably mammalian cells, are often provided by viral material. Commonly used promoters are derived from polyoma, adenovirus, and SV40. The early and late promoters of SV40 virus are particularly useful because both are obtained easily from the virus as a fragment that also contains the SV40 viral origin of replication (Fiers et al., Nature, 273,113; 1978). It is possible, and often desirable, to utilize promoter or control sequences normally associated with the desired gene sequence, provided such control sequences are compatible with the host cell systems.

An origin of replication may be provided either by construction of the vector to include an exogenous origin, such as may be derived from SV40 or other viral (e.g. Polyoma, Adeno, VSV, BPV) source, or may be provided by the host cell chromosomal replication mechanism. If the vector is integrated into the host cell chromosome, the latter is often sufficient.

The ligand may be a sugar, protein, peptide, or lipid.

### Ligand Template (VEGF)

Vascular endothelial growth factor (VEGF) is a secreted mitogen specific for vascular endothelial cells. VEGF induces angiogenesis and the permeabilization of blood vessels *in vivo*, as well as promotes endothelial cell proliferation and migration. VEGFs are secreted proteins in contrast to other endothelial cell mitogens such as acidic or basic fibroblast growth factors and platelet-derived endothelial cell growth factor. The secreted VEGFs stimulate endothelial cell proliferation by binding to specific tyrosine kinase receptors on the cell surface, known as vascular endothelial cell receptors (VEGF receptors). Binding of VEGF to its cognate receptor results in the ligand induced dimerization of the receptors, enabling the two cytoplasmic domains to cross-phosphorylate each other (autophosphorylate) on multiple tyrosine residues. Phosphorylation at these residues initiates an intracellular signaling cascade (presumably *via* transphosphorylation of specific downstream signal transduction protein mediators), setting into motion a series of intracellular reactions required for endothelial cell growth *via* the inhibition of apoptosis. In the absence of VEGF receptor mediated signaling, the endothelial cells undergo apoptosis.

VEGF is a glycosylated, disulfide-linked homodimeric protein consisting of two 23 kD subunits. Four different monomeric isoforms of VEGF exist ranging in size from 121 to 206 residues in humans (VEGF121, VEGF165, VEGF189 and VEGF206). Transcripts encoding the three shorter forms are detected in the majority of tumor cells and tumor tissue expressing VEGF gene. The isoforms result from different splicing events, and all variants share the same 115 N-terminal residues as well as six C-terminal residues. The leader sequence confers the ability of the VEGF molecule to exit the cells. VEGF165 is the dominant isoform, while VEGF206 has so far only been identified in human fetal liver cDNA library VEGF165 and VEGF189 bind heparin with high affinity, and are sequestered to the cell surface or within the extracellular matrix bound to proteoglycans, while VEGF121 does not bind heparin and is thus freely diffusible. Plasmin cleavage of VEGF165 generates a 110-residue long N-terminal fragment (the receptor-binding domain) that no longer binds heparin but is equipotent to VEGF121 in its ability to induce endothelial cell proliferation.

### C. Protein Localization Signals

It is well understood in the art that different types of signal peptides specify different locales within the cell. Proteins destined for initial transfer into the endoplasmic reticulum (ER) harbor an amino terminus signal peptide. From the ER, many of the proteins will continue on to the golgi apparatus, with the exception of those harboring a specific sequence of four amino acids at their carboxy terminus (retained in the ER). The proteins are then delivered to the plasma membrane by a "default" pathway, unless these proteins are diverted into other pathways or retained in specific compartments by sorting signals. Examples of sorting peptide signals include: H₃N-Gly-Ser-Ser-Lys-Ser-Lys-Pro-Lys (SEQ ID NO:1) (membrane attachment via the covalent linkage of a myristic acid to the amino terminus); H₃N-Met-Met-Ser-Phe-Val-Ser-Leu-Leu-Leu-Val-Gly-Ile-Leu-Phe-Trp-Ala-Thr-Glu-Ala-Glu-Gln-Leu-Thr-Lys-Cys-Glu-Val-Phe-Gln (SEQ ID NO:2) (import into the ER); Lys-Asp-Glu-Leu-COO- (SEQ ID NO:3) (retain in lumen of ER).

A signal sequence will often be necessary. Optionally, the ligand is expressed as a fusion with a plasma membrane anchor which prevents it from being completely secreted (once attached to the plasma membrane the expressed peptide is free to interact with the binding domain of, for example, the chimeric VEGF receptor). In general, the signal sequence may be a component of the retrovirus vector, or it may be a part of the desired protein DNA that is inserted into the vector. In some cases, the native desired protein DNA may encode a signal sequence at the amino terminus (5' end of the DNA) of the protein that is normally cleaved during post-translational processing of the protein to form the mature desired protein. Proteins may have the native signal sequence deleted and replaced with a heterologous signal sequence. The heterologous signal sequence selected should be one that is recognized and processed (i.e. cleaved by a signal peptidase) by the host cell. In mammalian cell expression the native signal sequence is satisfactory, although other mammalian signal sequences may be suitable.

### II. Ligand Characterization: Functional and Structural

The displayed peptide library may be analyzed to determine the diversity and/or composition of the amino acids incorporated. The cells displaying the ligand may be subjected to enzymes or acids known to specifically cleave between certain peptide residues to release the peptide of interest from the display chaperone protein (for example, formic acid cleaves peptide bonds between proline and glycine residues). The peptides are then hydrolyzed and analyzed for amino acid content using automated amino acid analyzers.

The peptides may also be analyzed for precise amino acid sequence. For example, the classic method of Edman degradation, in which the N-terminus of the peptide becomes modified, cleaved, and analyzed, thus shortening the peptide by one amino acid, is one way of extracting information at the amino acid level. Mass spectrometry is a more sophisticated technique and amenable to analyzing peptides that have incorporated amino acid analogues. Mass spectrometry utilizes helium gas to randomly cleave the peptide and subsequent analysis of the mass of the fragments generated are compared to elucidate the sequence. The peptide sequence can then be used to determine and/or design oligonucleotides encoding the peptides.

### Peptidic Activity

Receptor signaling is usually measured either phenotypically (for example, cell proliferation), or *via* an *in vitro* assay. Autophosphorylation assays may be employed to determine the level of enzymatic activity present in the chimeric receptor. In the autophosphorylation assay a chimeric fusion protein, containing the catalytic kinase domain and at least one affinity capture domain, is incubated in a buffer suitable for its enzymatic activity in the presence of radiolabeled ATP and ligand. The ligand bound kinase protein autophosphorylates resulting in the transfer of a radiolabeled phosphate group from ATP to the chimeric fusion protein. The radiolabeled receptor can be easily distinguished using common techniques, for example autoradiography. By modulating the phosphorylation of cell surface receptors, the ligands directly influence the activity of the cellular processes that these receptors control (many of which are easily observed or assayed).

Once a ligand has been identified as either altering the phosphorylation characteristics of the chimera receptor, or modulating some other enzymatic activity or phenotype associated with its binding, further studies may be incorporated to establish what effects post-translational, or peptide modification, may have on cell proliferation, cell growth and/or receptor autophosphorylation. Such modifications include, but are not limited to, site-directed mutagenesis, mutagenesis, acylation, acetylation, methylation, phosphorylation, sulfation, prenylation, glycosylation, carboxylation, ubiquitination, amidation, oxidation, hydroxylation, adding a seleno- group to amino acid side chains (for example, selenocysteine), and fluorescent labeling.

As discussed above, VEGF receptors, for example, play a critical role in cell growth by inhibiting apoptosis. Without proper VEGF receptor signaling, endothelial cells will undergo apoptosis. Frequently, apoptotic cells may be recognized by changes in their biochemical, morphological and/or molecular features. Morphological changes include, but are not limited to, cell shape change, cell shrinkage, cell detachment, apoptotic bodies, nuclear fragmentation, nuclear envelope changes and loss of cell surface structures. Biochemical changes may include proteolysis, protein cross linking , DNA denaturation, cell dehydration, intranucleosomal cleavage and a rise in free calcium ions. Such characteristics are easily identifiable by methods well established in the art.

When cells are no longer viable, i.e. they are dead, their membranes become permeabilized and this permeabilization will manifest itself as a change in the scattering of light. This scattering of light can be attributed to the change in the refractive index of the cell's cytoplasm. The use of DNA staining dyes that are able to pass through a permeabilized membrane, will aid in the identification of dead, live, and apoptotic cells. Flow cytometry and/or fluorescent activated cell sorting (FACS analysis) may be incorporated into protocols utilizing fluorescent dyes to separate the cells of interest. Flow cytometry can sort, or physically separate, particles of interest from a sample. Therefore, FACS analysis (which is a type of flow cytometry), may be defined as the physical separation of a cell or particle of interest from a heterogeneous population.

One may distinguish between dead, live, and apoptotic cells because each differ, for example, in their permeability to DNA dyes. Two widely used DNA dyes, Hoechst 33342 and propidium iodide (PI), are able to infiltrate dead cells. Live cells do not retain either dye, while apoptotic cells are able to retain Hoechst but not PI. Fluorescent microscopic observation will allow one to visually separate dead cells from live cells from cells undergoing apoptosis. Fluorescence emission from these different cells will also allow their separation via flow cytometry and/or FACS analyis. Typical stains used in these assays will include, propidium iodide, Hoechst 33342, 7AAD and TO-PRO-3.

Stages of membrane change during apoptosis may be analyzed as well. Among these changes is the translocation of phosphatidylserine (PS) from the inner part of the cell membrane to the outside during the early to intermediate stages of apoptosis. Using FITC labeled Annexin V, one may be able to detect PS. Annexin V is a Ca⁺⁺ dependent phospholipid-binding protein. Again, dead cells will not bind Annexin V. Live cells are also negative for Annexin Binding. Apoptotic cells bind Annexin. One may combine this method of analyzing PS with the aforementioned method of using PI to stain DNA, thereby obtaining different profiles of live, dead, and/or apoptotic cells.

As mentioned above, a characteristic of apoptosis is the degradation of DNA. This degradation is usually carried out by activated Ca/Mg dependent endonucleases. Terminal deoxynucleotidyl transferase (TdT) will add biotinylated, BrdU or digoxygenin-labeled nucleotides to DNA strand breaks. Subsequent binding of the exogenously added streptavidin by the biotin, or a fluorochrome labeled anti-digoxygenin antibody may be used to then detect DNA degradation. This method allows one to correlate apoptosis with cell cycle status.

Another DNA binding dye that may be incorporated is the laser dye styryl-751 (LDS-751). Again, one may take advantage of the ability of apoptotic cells to exhibit different staining patterns than that of live or dead cells.

Laser capture micro-dissection (LCM) is a relatively new technology used for the procurement of pure cells from various tissues. Isolated tissues may be used to identify what effects a peptide may have on cells that have either internalized the peptide or have bound the peptide to an outer surface receptor. After transfer film is applied to the surface of a particular tissue section, one may activate a pulsed laser beam that, in turn, activates the film immediately above the cell(s) of interest (morphological changes are easily identified and cells may be selected on this basis). The film melts and fuses the underlying cells. The film can then be removed and the remaining cells, not contained within the film, are left behind. Once the cells are isolated, DNA, RNA or protein from the cells may then be purified. The isolation of the cells *via* LCM does not damage the cells because the laser energy is absorbed by the film. This particular technology may be useful in combination with any of the previously mentioned methods of detecting proteins using fluorescent molecules.

Further *in vitro* analyses are used to study the effects of the peptides on cell viability. Peptides which either interrupt, stimulate, or decrease vital cellular processes may be used to infect cells, such as tumor cells, in culture. Once infected, cell growth and viability is analyzed by methods known in the art.

Many cells may undergo programmed cell death which is a genetically mediated form of self destruction (i.e. apoptosis; also described above). Apoptosis is an active suicide mechanism that is involved in normal tissue turnover during embryogenesis and adult life. Induction of apoptosis assures rapid disappearance of the immune response upon antigenic clearance, avoiding the metabolic costs involved in sustaining a large number of effector cells. Failure of immune cells to die is the cause of a number of immune-mediated disorders. (Agostini, C., et al. (1998) Curr. Opin. Pulm. Med. 4(5):261). Abnormal apoptotic activity has been implicated in a variety of diseases including cancer, autoimmunity, and degenerative disorders. (Phelps, et al. (2000) Endocrinol. Metab. Clin. North Amer. 29(2):375). Such autoimmune disorders include diabetes mellitus, systematic lupus erythematosus (SLE) and rheumatoid arthritis. Degenerative disorders include Parkinson's disease, Huntington's Chorea, Alzheimer's disease, and Pick's disease. Cancers include cancer of the bladder, brain cancer, breast cancer, colorectal cancer, hodgkins disease, cancer of the kidney, lung cancer, melanoma, non-hodgkins lymphoma, oral cancer, ovarian cancer, prostate cancer and uterine/cervical cancer.

*In vivo* analyses using animal models are used to determine the effects of the peptide within an intact system. For example, in the field of immunology, peptides can be administered to an animal and its peripheral blood monocytes are used in the generation of antibodies directed against the peptide.

In the case of viral proteins --for use with, for example, viral vectors, therapeutic viruses, and viral capsid delivery compositions -- desired characteristics to be retained can include the ability to assemble into a viral particle or capsid and the ability to infect or enter cells. Such characteristics are useful where the delivery properties of the viral proteins are of interest.

One application of the disclosed method is in the identification and development of peptides, and the oligonucleotides encoding those peptides, for use in subsequent gene replacement and/or gene enhancement therapy. For example, identifying anti-tumor peptides that specifically target the receptors involved in the metastatic spread of tumors.

It may also be desirable to use a constructed endothelial cell line which expresses a chimeric VEGF receptor to screen conditioned media prepared from a variety of cultured cell lines, or media containing any peptides or molecules of interest, to identify proteins, peptides, or molecules that support proliferation or growth of, for example, the VEGF- signaling dependent cells. Media containing ligand mitogenic activity may then be tested at various dilutions to establish a dose-dependent proliferation effect. In addition, control assays with, for example, neutralizing antibodies, are performed. The conditioned media that appear positive may be adsorbed using soluble receptor, or soluble receptor binding domain, to remove the proliferative activity. The cell lines that produce the conditioned medium with proliferative activity are used to prepare RNA/DNA for construction of expression libraries according to methods known in the art (see e.g., Chapters 5 and 6 in Current Protocols in Molecular Biology, F.M. Ausubel et al., John Wiley & Sons, Boston, Mass. (1994), pp. 5.0.1-6.12.12). Further screening of these expression libraries can be performed as described above. Alternatively, classical protein purification techniques can be performed to isolate ligands from positive conditioned media (see e.g., Chapter 10 in Current Protocols in Molecular Biology, F.M. Ausubel et al., eds., John Wiley & Sons, Boston, Mass. (1994) pp. 10.0.1-10.19.12).

Once a cDNA encoding a putative ligand has been isolated, the protein encoded by that cDNA is produced and characterized with respect to binding to the displayed receptor. Further, as described above, the biological effects of the ligand on chimeric receptor-positive cells are determined and further characterized.

### III. Pharmaceutical Compositions

Any of the peptides, or derivatives thereof, obtained using this method, as disclosed herein, can be incorporated into pharmaceutical compositions suitable for administration. Such compositions typically comprise the nucleic acid molecule, protein, peptide, or antibody to be delivered and a pharmaceutically acceptable carrier. As used herein the term "pharmaceutically acceptable carrier" is intended to include any and all solvents, dispersion, media, coatings, antibacterial an antifungal agents, and isotonic and absorption delaying agents, compatible with pharmaceutical administration. The use of such media and agents for pharmaceutically active substances is well known in the art. Additional active compounds can also be incorporated into the compositions.

The peptides can be administered to a patient or cells of a patient, in need thereof, for example, in an effective amount for treatment of cancer or proliferative disorders, in a pharmaceutically acceptable carrier such as saline, polymer or liposomal carriers, or enteric coated tablets or capsules. Other exemplary uses are shown by the following examples.

The pharmaceutical composition is formulated to be compatible with its intended route of administration. Examples of routes of administration include parenteral, e.g. intravenous, intradermal, subcutaneous, inhalation, transmucosal, and rectal administration; enteral (oral); and transdermal or topical. Solutions or suspensions suitable for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials. Pharmaceutical compositions suitable for injection typically include sterile aqueous solutions (where water soluble) or dispersions and sterile powders for the extemporaneous preparation of sterile injectable solutions or dispersions. In all cases, the composition must be sterile. It must be stable under the conditions of manufacture and storage and must be preserved against the contaminating action of microorganisms such as bacteria and fungi.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. The active compound can be incorporated with excipients and used in the form of tablets, troches, or capsules. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel^{™}, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds/peptides are delivered in the form of an aerosol spray from pressured container or dispenser which contains a suitable propellant, *e.g.*, a gas such as carbon dioxide, or a nebulizer, or as a dry powder.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. The compounds/peptides can also be prepared in the form of suppositories (*e.g.*, with conventional suppository bases such as cocoa butter and other glycerides) or retention enemas for rectal delivery.

In one embodiment, the active compounds/peptides are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms is dictated by the unique characteristics of the active compound and the particular therapeutic effect to be achieved, and the limitations inherent in the art of compounding such an active compound for the treatment of individuals.

Toxicity and therapeutic efficacy of such compounds/peptides can be determined by standard pharmaceutical procedures in cell cultures or experimental animals, *e.g.,* for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. The therapeutically effective dose can be estimated initially from cell culture assays. A dose may be formulated in animal models to achieve a circulating plasma concentration range that includes the IC50 (*i*.*e*., the concentration of the test compound which achieves a half-maximal inhibition of symptoms) as determined in cell culture. Such information can be used to accurately determine useful doses in humans. Levels in plasma may be measured, for example, by high performance liquid chromatography.

For delivery of nucleic acid molecules, the molecules are typically inserted into gene therapy vectors. Gene therapy vectors can be delivered to a subject by, for example, intravenous injection or local administration. Alternatively, where the complete gene delivery vector can be produced intact from recombinant cells, *e.g.,* retroviral vectors, the pharmaceutical preparation can include one or more cells which produce the gene delivery system.

The present invention will be further understood by reference to the following hypothetical examples.

### Example 1: Enhancing Hematopoiesis by Targeting the Stem Cell Factor Receptor (SCF receptor, or Kit)

The SCF receptor plays important roles in regulating the growth, survival and migration of hematopoietic progenitor cells. Activating SCF receptor, which acts in synergy with the EPO receptor, results in an easily assayable phenotype. Thus, peptides that stimulate signaling through the SCF receptor may augment EPO signaling and hence, erythropoiesis. Mice harboring loss of function mutations in either SCF receptor, or SCF develop severe anemias, thereby illustrating the essential role for these proteins in eythropoiesis. Moreover, SCF receptor and EPO receptor co-function in striking synergy to promote erythroid progenitor cell production.

The chimeric receptor approach is utilized to isolate and characterize molecules, compounds and proteins that modulate SCF receptor and EPO receptor function, thereby modulating hematopoeisis (i.e. using SCF receptor ligand binding domain or the EPO receptor ligand binding domain fused to an output signaling domain - analogous to intracellular signaling domain).

### Example 2: Enhancing Hematopoiesis through Activation of VIP-1 (Vasso-Active Intestinal Peptide) Receptor

Activation of the VIP-1 receptor has been shown to augment EPO signaling. Several compounds have already been identified which can stimulate VIP-1 receptor. Upon VIP-1 receptor stimulation, PI3 kinase and protein kinase C become activated. In recent *in vivo* studies, these compounds triple the red blood cell count in treated animals. Further studies incorporate the use of a chimeric receptor protein harboring the VIP-1 ligand binding domain.

### Example 3: Targeting G-CSF (Granulocyte-Colony Stimulating Factor)

The production of neutrophils and monocytes from hematopoietic stem cells is governed by a family of hematopoietic growth factors. G-CSF (granulocyte-colony stimulating factor) and GM-CSF (granulocyte/macrophage stimulating factor) are potent stimulators of the early phases of leukocyte productin, acting through specific receptors on the surface of the hematopoietic cells. Physiological studies show that G-CSF levels increase in the blood when blood neutrophil levels fall to extremely low levels. Animal studies have shown that G-CSF is the critical regulator of neutrophils in the blood. Mature neutrophils and monocytes bear receptors for both G-CSF and GM-CSF, and the functions of these cells are enhanced by exposure to the cytokines. Therapy involving the transplantation of early hematopoietic progenitors has been successful for a variety of malignant and inherited diseases, and provides myelopoietic support for patients undergoing high-does chemotherapy or radiotherapy. However, stem or progenitor cell transplantation has been limited by A) the need for a sufficient quantity of stem cells to achieve benefit and B) mature blood cell regeneration after transfusion is slow, requiring one to three weeks.

Drug compounds for G-CSF mimetics have been identified and stimulate signaling through the G-CSF receptor domain of a chimeric receptor. The compounds have one or more of the following characteristics: stable at room temperature, resistant to proteolytic cleavage, have long circulating half life, require less dosing frequency, do not elicit antibodies against G-CSF, and target only G-CSF responsive cells. Such compounds can be readily assayed and further characterized using chimeric receptors containing a G-CSF receptor domain.

## Claims

1. A method for identifying a ligand comprising: (a) expressing a fusion ligand in a eukaryotic cell host comprising a plasma membrane, wherein the fusion ligand is encoded by a chimeric gene comprising: a DNA fragment encoding a peptide which mediates attachment of the fusion ligand to the plasma membrane, and a DNA fragment encoding a ligand; (b) contacting the fusion ligand of step (a) with a chimeric receptor expressed in the same host cell, wherein the chimeric receptor comprises, an intracellular domain fused to an extracellular ligand binding domain; (c) identifying those cells that exhibit a change in phenotype; and (d) identifying the ligand.

2. The method of claim 1 further comprising: (e) isolating the cell host.

3. The method of claim 2 further comprising: (f) isolating the ligand of step (a).

4. The method of claim 1, wherein the extracellular domain is indirectly fused to the intracellular domain by a transmembrane domain or peptide linker.

5. The method of claim 1, wherein the extracellular ligand binding domain is selected from the group consisting of EPO receptor ligand binding domain, VEGF receptor ligand binding domain, Flt-1 receptor ligand binding domain, KDR receptor ligand binding domain, Neuropilin receptor ligand binding domain, Flt-4 receptor ligand binding domain, and VIP-1 receptor ligand binding domain.

6. The method of claim 1, wherein the intracellular domain is selected from the group consisting of a kinase domain; a dimerization domain; a kinase and dimerization domain; a protein binding domain; and a kinase and dimerization and protein binding domain.

7. The method of claim 1, wherein DNA fragment encoding a peptide which mediates attachment of the fusion ligand to the plasma membrane encodes H₃N-Gly-Ser-Ser-Lys-Ser-Lys-Pro-Lys (SEQ ID NO:1).

8. The method of claim 1, wherein the host eukaryotic cell is selected from the group consisting of a plant cell, a mammalian cell, and a yeast cell.

9. The method of claim 8, wherein the mammalian cell is an endothelial cell.

10. The method of claim 1, wherein the DNA fragment that encodes the peptide mediating attachment of the fusion protein to the plasma membrane contains a signal amino acid sequence.

11. The method of claim 10, wherein the signal sequence contains lipid modification sites.

12. The method of claim 1, wherein the DNA fragment encoding the ligand is from a DNA or cDNA library.

13. The method of claim 3 further comprising: (g) isolating the DNA encoding the fusion ligand; and (h) subjecting the isolated DNA to analysis methods selected from the group consisting of determining DNA base composition, determining DNA base sequence, determining molecular weight, and determining secondary structures within the sequence.

14. The method of claim 1 wherein the DNA encoding the ligand fusion and the chimeric receptor is produced from the RNA introduced into the eukaryotic cell by infection with a retrovirus.

15. A peptide identified using the method of claim 1.

16. The peptide of claim 15, wherein the peptide is identified as a ligand selected from the group consisting of VEGF receptor ligand and an EPO receptor ligand.

17. An oligonucleotide library for screening using the method of claim 1.

18. The peptide of claim 15 further comprising a pharmaceutically acceptable carrier for administration to a patient.

19. A method of use of the peptide formulation of claim 18 to treat a patient.

20. The method of claim 19, wherein the patient has cancer.

21. The method of claim 19, wherein the patient has an autoimmune disorder.

22. The method of claim 19, wherein the patient has a degenerative disorder.

23. The method of claim 20, wherein the cancer is selected from the group consisting of bladder cancer, brain cancer, breast cancer, colorectal cancer, hodgkins disease, cancer of the kidney, lung cancer, melanoma, non-hodgkins lymphoma, oral cancer, ovarian cancer, prostate cancer and uterine/cervical cancer.

24. The method of claim 21, wherein the autoimmune disorder is selected from the group consisting of diabetes mellitus, systematic lupus erythematosus (SLE) and rheumatoid arthritis.

25. The method of claim 22, wherein the degenerative disorder is selected from the group consisting of Parkinson's disease, Huntington's Chorea, Alzheimer's disease, and Pick's disease.

26. A peptide isolated by the method of claim 1, wherein the receptor fusion contains a G-CSF receptor domain.

27. A method for identifying a ligand comprising: (a) expressing a fusion ligand in a eukaryotic cell host comprising a plasma membrane, wherein the fusion ligand is encoded by a chimeric gene comprising: a DNA fragment encoding a peptide which mediates attachment of the fusion ligand to the plasma membrane, and a DNA fragment encoding a ligand; (b) contacting the fusion ligand of step (a) with a chimeric receptor on the same host cell, wherein the chimeric receptor comprises, an intracellular domain fused to an extracellular ligand binding domain; (c) identifying those cells that exhibit a phenotype; and (d) identifying the ligand.

28. The method of claim 27 further comprising: (e) isolating the cell host.

29. The method of claim 28 further comprising: (f) isolating the ligand of step (a).

30. The method of claim 27, wherein the extracellular domain is indirectly fused to the intracellular domain by a transmembrane domain or peptide linker.

31. The method of claim 27, wherein the extracellular ligand binding domain is selected from the group consisting of EPO receptor ligand binding domain, VEGF receptor ligand binding domain, Flt-1 receptor ligand binding domain, KDR receptor ligand binding domain, Neuropilin receptor ligand binding domain, Flt-4 receptor ligand binding domain, and VIP-1 receptor ligand binding domain.

32. The method of claim 27, wherein the intracellular domain is selected from the group consisting of a kinase domain; a dimerization domain; a kinase and dimerization domain; a protein binding domain; and a kinase and dimerization and protein binding domain.

33. The method of claim 27, wherein DNA fragment encoding a peptide which mediates attachment of the fusion ligand to the plasma membrane encodes H3N-Gly-Ser-Ser-Lys-Ser-Lys- Pro-Lys (SEQ ID NO:1).

34. The method of claim 27, wherein the host eukaryotic cell is selected from the group consisting of a plant cell, a mammalian cell, and a yeast cell.

35. The method of claim 34, wherein the mammalian cell is an endothelial cell.

36. The method of claim 27, wherein the DNA fragment that encodes the first peptide mediating attachment of the fusion protein to the plasma membrane contains a signal amino acid sequence.

37. The method of claim 36, wherein the signal sequence contains lipid modification sites.

38. The method of claim 27, wherein the DNA fragment encoding the ligand is from a DNA, cDNA, or RNA library.

39. The method of claim 29 further comprising: (g) isolating the DNA encoding the fusion ligand; and (h) subjecting the isolated DNA to analysis methods selected from the group consisting of determining DNA base composition, determining DNA base sequence, determining molecular weight, and determining secondary structures within the sequence.

40. The method of claim 27 wherein the DNA encoding the ligand fusion and the chimeric receptor is produced from the RNA introduced into the endothelial cell by infection with a retrovirus.

41. A method for identifying a ligand comprising: (a) expressing a ligand in a cell host; (b) contacting the ligand of step (a) with a chimeric receptor in the same cell host, wherein the chimeric receptor comprises, an intracellular signaling domain fused to, an intracellular ligand binding domain, thereby resulting in a cytoplasmic chimeric receptor; and (c) identifying those cells that exhibit a phenotype; and (d) identifying the ligand.

42. The method of claim 41 further comprising: (e) isolating the cell host.

43. The method of claim 42 further comprising: (f) isolating the ligand of step (a).

44. A peptide identified using the method claim of 27 or claim 41.

45. The peptide of claim 44, wherein the peptide is identified as a ligand selected from the group consisting of VEGF receptor ligand and an EPO receptor ligand.

46. An oligonucleotide library for screening using the method of claim 27 or claim 41.

47. The peptide of claim 44 further comprising a pharmaceutically acceptable carrier for administration to a patient.

48. A method of use of the peptide formulation of claim 47 to treat a patient.

49. The method of claim 48, wherein the patient has cancer.

50. The method of claim 48, wherein the patient has an autoimmune disorder.

51. The method of claim 48, wherein the patient has a degenerative disorder.

52. The method of claim 49, wherein the cancer is selected from the group consisting of bladder cancer, brain cancer, breast cancer, colorectal cancer, hodgkins disease, cancer of the kidney, lung cancer, melanoma, non-hodgkins lymphoma, oral cancer, ovarian cancer, prostate cancer and uterine/cervical cancer.

53. The method of claim 50, wherein the autoimmune disorder is selected from the group consisting of diabetes mellitus, systematic lupus erythematosus (SLE) and rheumatoid arthritis.

54. The method of claim 51, wherein the degenerative disorder is selected from the group consisting of Parkinson's disease, Huntington's Chorea, Alzheimer's disease, and Pick's disease.

55. A peptide isolated by the method of claim 27 or claim 43, wherein the receptor fusion contains a G-CSF receptor domain.

56. The method of claim 41, wherein the DNA fragment encoding the ligand is from a DNA, cDNA, or RNA library.

57. The method of claim 43 further comprising: (g) isolating the DNA encoding the fusion ligand; and (h) subjecting the isolated DNA to analysis methods selected from the group consisting of determining DNA base composition, determining DNA base sequence, determining molecular weight, and determining secondary structures within the sequence.

58. The method of claim 41 wherein the DNA encoding the ligand fusion and the chimeric receptor is produced from the RNA introduced into the endothelial cell by infection with a retrovirus.


SEQUENCE LISTING

<110> Sopherion Therapeutics, Inc.

<120> Methods to screen ligands using eukaryotic cell display

<130> JEC/FP6500938

<140> Divisional of EP 03736907.1
<141> 2003-06-05

<140> EP 03736907.1
<141> 2003-06-05

<140> PCT/US03/17949
<141> 2003-06-05

<150> 60/386,584
<151> 2002-06-05

<160> 3

<170> PatentIn version 3.1

<210> 1
<211> 8
<212> PRT
<213> Artificial sequence

<220>
<223> Eukaryotic protein sorting signal

<400> 1

Gly Ser Ser Lys Ser Lys Pro Lys
1               5


<210> 2
<211> 29
<212> PRT
<213> Artificial sequence

<220>
<223> Eukaryotic protein sorting signal

<400> 2

Met Met Ser Pro Val Ser Leu Leu Leu Val Gly Ile Leu Phe Trp Ala
1               5                   10                  15


Thr Gly Ala Glu Gln Leu Thr Lys Cys Glu Val Phe Gln
            20                  25


<210> 3
<211> 4
<212> PRT

<213>  Artificial sequence

<220>
<223>  Eukaryotic protein sorting signal

<400>  3

Leu Pro Glu Leu
1

SEQUENCE LISTING

<110>  Sopherion Therapeutics, Inc.

<120>  Method to screen ligands using eukaryotic cell display

<130>  SOPH 100 PCT

<150>  60/386,584
<151>  2002-06-05

<160>  3

<170>  PatentIn version 3.1

<210>  1
<211>  8
<212>  PRT
<213>  Artificial sequence

<220>
<223>  Eukaryotic protein sorting signal

<400>  1

Gly Ser Ser Lys Ser Lys Pro Lys
1               5


<210>  2
<211>  29
<212>  PRT
<213>  Artificial sequence

<220>
<223>  Eukaryotic protein sorting signal

<400>  2

Met Met Ser Pro Val Ser Leu Leu Leu Val Gly Ile Leu Phe Trp Ala
1               5                   10                  15


Thr Gly Ala Glu Gln Leu Thr Lys Cys Glu Val Phe Gln
            20                  25


<210>  3
<211>  4
<212>  PRT
<213>  Artificial sequence

<220>
<223>  Eukaryotic protein sorting signal

<400>  3

Leu Pro Glu Leu
1